# EUROPEAN PATENT APPLICATION

(11) **EP 4 648 055 A1**
(43) Date of publication of application: **12.11.2025**
(21) Application number: 25172995.0
(22) Date of filing: 28.04.2025
(51) Int. Cl.: G16H 10/40, G01N 35/00, G06Q 10/0631, G16H 40/20, G05B 19/418

(54) **METHOD FOR CONFIGURING PIPELINE SCHEDULING STRATEGY, PIPELINE CONTROL TERMINAL, AND SYSTEM**

(30) Priority: 11.05.2024 CN 202410600654
(71) Applicant: Shenzhen New Industries Biomedical Engineering Co., Ltd., Shenzhen, Guangdong 518122 (CN)
(72) Inventor: CHEN, Shujuan, Shenzhen (CN); PAN, Li, Shenzhen (CN); TANG, Junhui, Shenzhen (CN); DENG, Xiaoting, Shenzhen (CN); MA, Xie, Shenzhen (CN); ZHU, Xiaoyang, Shenzhen (CN); CHEN, Hua, Shenzhen (CN); XU, Baixun, Shenzhen (CN); DENG, Yinghua, Shenzhen (CN)
(74) Representative: Office Freylinger

(57) **Abstract**

This application relates to a method for configuring a pipeline scheduling strategy, a pipeline control terminal, and a pipeline system. The method includes: determining a processing content of a scheduling strategy; the processing content of the scheduling strategy including a target function determined from processing functions provided by a pipeline; determining a target sample applying the scheduling strategy; and configuring the scheduling strategy according to the processing content and the target sample, where the scheduling strategy is used to instruct a processing module with the target function on the pipeline to process the target sample. The method can flexibly specify different processing methods for different samples on the pipeline, thereby enhancing an intelligence level of pipeline scheduling.

## Description

### Technical Field

This application relates to the technical field of medical laboratory testing and sample analyzers, and in particular, to a method for configuring a pipeline scheduling strategy, a pipeline control terminal, and a pipeline system.

### Background

In the field of medical laboratory testing, a preprocessing module, a sample analyzer, and a post-processing module can be connected through a track according to requirements. Then, through a pipeline control terminal, functions such as data integration, process control, automatic operations, and data analysis are achieved, thereby more efficiently and automatically processing and analyzing biological samples.

A conventional pipeline control terminal can determine strategies according to sample features, priorities, instrument status, load, and other factors, such as priority scheduling and load balancing scheduling. However, these scheduling strategies are relatively conventional and cannot satisfy the requirements of users for intelligent scheduling.

### Summary

Based on this, for the above technical problems, it is necessary to provide a method and apparatus for configuring a pipeline scheduling strategy, a pipeline control terminal, a pipeline system, and a computer-readable storage medium, that can satisfy intelligent scheduling requirements.

A method for configuring a pipeline scheduling strategy includes:
determining a processing content of a scheduling strategy, where the processing content of the scheduling strategy includes a target function determined from processing functions provided by a pipeline;
determining a target sample applying the scheduling strategy; and
configuring the scheduling strategy according to the processing content and the target sample, where the scheduling strategy is used to instruct a processing module with the target function on the pipeline to process the target sample.

An apparatus for configuring a pipeline scheduling strategy includes:
a function determination component, configured to determine a processing content of a scheduling strategy, where the processing content of the scheduling strategy includes a target function determined from processing functions provided by a pipeline;
an object determination component, configured to determine a target sample applying the scheduling strategy; and
a configuration component, configured to configure the scheduling strategy according to the processing content and the target sample, where the scheduling strategy is used to instruct a processing module with the target function on the pipeline to process the target sample.

A pipeline control terminal includes a memory and a processor. The memory has a computer program stored therein. The processor, when executing the computer program, implements the steps of the above method for configuring a pipeline scheduling strategy.

A pipeline system includes a track, various function processing modules connected through the track, and the above pipeline control terminal. The processing modules and the track are respectively in communication connection with the pipeline control terminal.

A computer-readable storage medium has a computer program stored therein. The computer program, when executed by a processor, implements the steps of the above method for configuring a pipeline scheduling strategy.

According to the method and apparatus for configuring a pipeline scheduling strategy, the pipeline control terminal, the pipeline system, and the computer-readable storage medium, by configuring the processing content of the scheduling strategy and the target sample applying the scheduling strategy, different processing methods can be flexibly specified for different samples on the pipeline, thereby enhancing an intelligence level of pipeline scheduling.

### Brief Description of the Drawings

Fig. 1 is a schematic diagram of a structure of a pipeline system according to an embodiment;
Fig. 2 is a schematic flowchart of a method for configuring a pipeline scheduling strategy according to an embodiment;
Fig. 3 is a schematic diagram of a processing content configuration interface according to an embodiment;
Fig. 4 is a schematic diagram of an application object configuration interface according to an embodiment;
Fig. 5 is a schematic diagram of a scheduling strategy preview interface according to an embodiment;
Fig. 6 is a block diagram of a structure of an apparatus for configuring a pipeline scheduling strategy according to an embodiment; and
Fig. 7 is a diagram of an internal structure of a pipeline control terminal according to an embodiment.

### Detailed Description of the Embodiments

In order to provide a clearer understanding of the objectives, technical solutions, and advantages of this application, this application is further described in detail in conjunction with accompanying drawings and embodiments below. It should be understood that the specific embodiments described herein are merely used to explain this application rather than limit this application.

In the field of medical laboratory testing, a preprocessing module, a sample analyzer, and a post-processing module can be connected through a track according to requirements. Then, through a pipeline control terminal, functions such as data integration, process control, automatic operations, and data analysis are achieved, thereby more efficiently and automatically processing and analyzing biological samples. The preprocessing module may include a sample feed processing module, a centrifugation processing module, an uncovering processing module, etc., which respectively achieve functions such as sample feed, centrifugation, and uncovering. The sample analyzer may be configured with a biochemical analyzer, an immunoassay analyzer, etc., according to testing requirements, to achieve the function of analyzing different test items of samples. The post-processing module may include a film sealing processing module, a sample discharge processing module, etc., which are used for achieving functions such as film sealing and sample discharge.

A conventional pipeline control terminal can determine strategies according to sample features, priorities, instrument status, load, and other factors, such as priority scheduling and load balancing scheduling. However, these scheduling strategies are relatively conventional and cannot satisfy the requirements of users for intelligent scheduling.

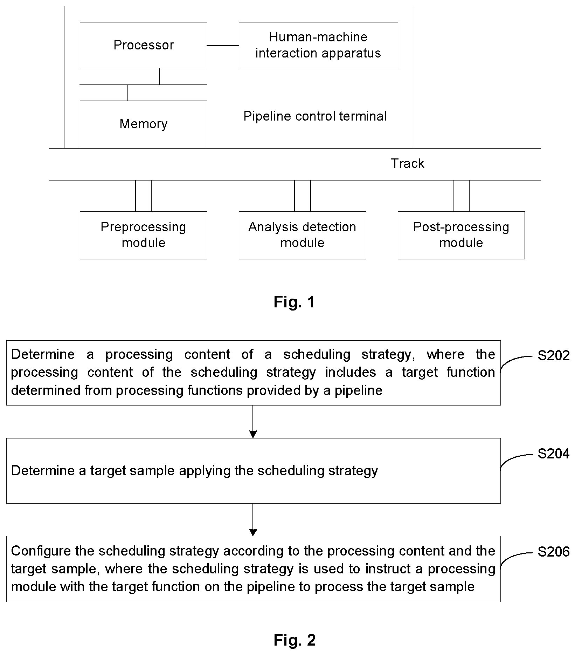

To solve the problems, this application provides a pipeline system, as shown in Fig. 1, including a track, various function processing modules connected through the track, and a pipeline control terminal. The processing modules and the track are respectively in communication connection with the pipeline control terminal. The processing modules may include a preprocessing module, an analysis detection module, and a post-processing module. The pipeline control terminal includes a memory and a processor. The memory has a computer program stored therein. The processor, when executing the computer program, implements the steps of a method for configuring a pipeline scheduling strategy. For easy user configuration, the pipeline control terminal also includes a human-machine interaction apparatus connected with the processor. The human-machine interaction apparatus may provide a configuration interface to facilitate a user in scheduling strategy configuration.

The pipeline control terminal is responsible for managing and coordinating a processing order of each instrument and step in a sample analyzer pipeline, and ensuring that a series of operations from sample feed to sample discharge can be carried out efficiently and orderly.

To satisfy the requirements of the user for intelligent scheduling, this application provides a method for configuring a pipeline scheduling strategy, applicable to the pipeline control terminal as shown in Fig. 1. As shown in Fig. 2, the method includes the following steps:

Step 202: Determine a processing content of a scheduling strategy, where the processing content of the scheduling strategy includes a target function determined from processing functions provided by a pipeline.

Alternatively, the target function is selected from the processing functions provided by the pipeline to serve as the processing content of the scheduling strategy, so that the processing content represents a processing task to be performed by the scheduling strategy. For example, centrifugation, uncovering, and classified sample discharge may be selected from the processing functions provided by the pipeline as the processing content of the scheduling strategy, meaning that the tasks to be processed by the scheduling strategy include centrifugation, uncovering, and classified sample discharge.

By determining the processing content of the scheduling strategy, scheduling a target sample to which processing modules for which processing can be determined on the pipeline through the scheduling strategy, thereby achieving corresponding functions.

In some embodiments, in response to configuring the target function of the scheduling strategy, the configuration is completed by selecting the arrangement of the processing module with the target function. However, to improve the processing efficiency of the pipeline, a plurality of functions may be integrated onto a single instrument or module on the pipeline. For example, the uncovering function and a serum quality testing function are integrated into one module for processing. After a sample tube enters the processing module, serum quality testing is performed first, and then uncovering processing is performed. If the scheduling strategy is configured according to the processing module with the target function, the scheduling strategy can only be configured for execution of all functions of an integrated module without personalized selection of some of functions of the integrated module.

In some embodiments, the target function of the scheduling strategy is determined from the processing functions provided by the pipeline. The plurality of functions of the integrated module is decomposed to facilitate user selection. For example, the uncovering function may be selected from one configuration strategy, or the serum quality testing function may be selected from one configuration strategy, thereby achieving personalized selection of some of the functions of the integrated module.

Step 204: Determine a target sample applying the scheduling strategy.

Alternatively, the target sample applying the scheduling strategy is determined, namely determining an application object for the scheduling strategy. By configuring the target sample applying the scheduling strategy, which samples on the pipeline that need to apply the scheduling strategy can be determined.

In some embodiments, the setting may be performed by limiting conditions of the target sample, and the sample that meets the conditions is determined as the target sample, thereby implementing the scheduling strategy to the target sample. In some embodiments, if the conditions of the target sample are not set, the scheduling strategy may be implemented to all samples on the pipeline.

Step 206: Configure the scheduling strategy according to the processing content and the target sample, where the scheduling strategy is used to instruct the processing module with the target function on the pipeline to process the target sample.

In some embodiments, to facilitate operations, a configuration interface may be provided to the user, to facilitate the user to set the processing content of the scheduling strategy and the target sample applying the scheduling strategy. After the user clicks a "save" button on the configuration interface, the scheduling strategy can be configured according to the processing content and the target sample. According to the scheduling strategy, the pipeline control terminal instructs the processing module with the target function on the pipeline to process the target sample.

For example, the processing content of the scheduling strategy is the classified sample discharge, the target sample applying the scheduling strategy is a target sample that meets a condition B, and therefore according to the scheduling strategy, the sample discharge processing module is instructed to perform sample discharge processing on the target sample meeting the condition B according to a classified sample discharge rule.

In some embodiments, scheduling strategies that the user can configure are diverse. For ease of management, in response to configuring a scheduling strategy, the user may also configure a name to each scheduling strategy to facilitate the management of the scheduling strategy.

In some embodiments, by configuring the processing content of the scheduling strategy and the target sample applying the scheduling strategy, different processing methods can be flexibly specified for different samples on the pipeline, thereby enhancing an intelligence level of pipeline scheduling.

In some embodiments, the processing content includes at least two target functions, and the processing content further includes: a processing order of the at least two target functions.

The step of configuring the scheduling strategy according to the processing content and the target sample includes: configuring the scheduling strategy according to the target functions, the processing order of the target functions, and the target sample, where the scheduling strategy is used to instruct, according to the processing order, sequential scheduling of the target sample to the processing modules with the target functions on the pipeline for processing.

In some embodiments, in response to needing to configure the plurality of target functions for the processing content configured by the scheduling strategy, the processing order of the plurality of target functions needs to be further configured. It should be understood that the processing order of the target functions should be limited by a processing order of functions on the pipeline. For example, according to the processing order of the functions on the pipeline, the uncovering function should be performed after a centrifugation function, and therefore during configuring the processing content of the scheduling strategy, the uncovering function is not allowed to be configured before the centrifugation function, in response to the plurality of target functions including the uncovering function and the centrifugation function.

Correspondingly, the scheduling strategy is configured according to the target functions, the processing order of the target functions, and the target sample, where the scheduling strategy is used to instruct, according to the processing order, sequential scheduling of the target sample to the target function processing modules on the pipeline for processing.

For example, the processing content of the scheduling strategy includes centrifugation, uncovering, and sample discharge, the target sample applying the scheduling strategy is a target sample that meets a condition A, and therefore according to the scheduling strategy, the target sample that meets the condition A is sequentially scheduled to the centrifugation processing module, the uncovering processing module, and the sample discharge processing module respectively for centrifugation processing, uncovering processing, and sample discharge processing.

In some embodiments, in response to needing to configure the plurality of target functions need to be configured for the scheduling strategy, the processing order of the plurality of modules with the target functions is further configured, to satisfy diverse configuration requirements of the scheduling strategy and increase the intelligent level of pipeline scheduling.

In some embodiments, the step of determining a processing content of a scheduling strategy includes: providing a processing content configuration interface, where the processing content configuration interface includes at least one setting component, and the setting component at least includes a setting bar; and in response to a target function set at the setting bar of the first or last setting component being a classified sample discharge function, obtaining the processing content of the scheduling strategy according to the classified sample discharge function, where the scheduling strategy is used to instruct the sample discharge processing module to perform sample discharge processing on the target sample according to the classified sample discharge rule.

In some embodiments, by providing the processing content configuration interface, the user operation is facilitated for configuring the processing content of the scheduling strategy. Alternatively, the processing content configuration interface is provided with the at least one setting component, the setting component at least includes the setting bar, and the user may set the target function module through the setting bar.

According to the processing order of the functions of the pipeline, the sample discharge function is the last processing function in the pipeline. The sample discharge function refers to a process of sequentially removing samples from the pipeline and placing them into sample boxes for storage after the samples have been analyzed and processed in the pipeline.

With the intelligent development of the pipeline, the user puts forward numerous requirements for sample discharge methods. To satisfy the user-related sample discharge requirements, the classified sample discharge method is provided in the pipeline. Classified sample discharge refers to placing samples of specified types into sample boxes of specified types according to a preset classification rule during sample discharge. Alternatively, the sample discharge processing module of the sample analyzer may set types of sample boxes in a sample discharge drawer, thereby achieving separate storage of the samples of the specified types to different types of sample boxes.

According to the classified sample discharge method, a classified sample discharge rule needs to be pre-configured. The classified sample discharge rule includes a placement position of each type of samples in the sample discharge drawer, with each placement position corresponding to the type of the sample boxes in the sample discharge drawer. For example, an A-type sample box and a B-type sample box are arranged in the sample discharge drawer. The classified sample discharge rule includes placing samples for an A test item to the A-type sample box of the sample discharge drawer, and placing samples for a B test item to the B-type sample box of the sample discharge drawer.

In response to setting the classified sample discharge function in the setting bar of the first setting component by the user, since sample discharge is the last processing function in the pipeline, in response to setting the classified sample discharge function in the setting bar of the first setting component by the user, the processing content of the scheduling strategy can be obtained according to the classified sample discharge function. The processing content of the scheduling strategy is the classified sample discharge function. The corresponding scheduling strategy is used to instruct the sample discharge processing module to perform sample discharge processing on the target sample according to the classified sample discharge rule.

The scheduling strategy may be applied to scenarios where a doctor needs to classify samples. In response to a large number of samples requiring manual classification by the doctor, the classified sample discharge function of the pipeline may be used, and after the classified sample discharge rule is configured, these samples are placed at a sample feed position, and then the user configures a scheduling strategy. The configuration of the scheduling strategy includes configuring the classified sample discharge function at the setting bar of the first setting component through the processing content configuration interface, and setting the sample feed position. The pipeline directly instructs the sample discharge processing module to perform sample discharge processing on the samples at the sample feed position according to the classified sample discharge rule. No manual classification is needed in the process, thereby improving the processing efficiency.

In response to setting the classified sample discharge function at the setting bar of the last setting component by the user, the scheduling strategy includes a target function set in a previous setting component and the classified sample discharge function set in the last setting component, and the scheduling strategy is used to instruct the corresponding processing module of the pipeline to perform the previous target function on the target sample and then instruct the sample discharge processing module to perform sample discharge processing on the target sample according to the classified sample discharge rule. Therefore, the samples can be processed according to the classified sample discharge rule during sample discharge, and after a series of function processing on the samples is completed, the different types of samples are respectively stored in the different types of sample boxes.

To achieve diversity of the scheduling strategy and intelligent scheduling, this application may further provide a method for configuring diverse scheduling strategies. Alternatively, the step of determining a processing content of a scheduling strategy includes: providing a processing content configuration interface, where the processing content configuration interface includes at least one setting component, and the setting component at least includes a setting bar; in response to acquiring a plurality of target functions set at the setting bars of the plurality of setting components, acquiring a processing order of the target functions; and obtaining the processing content of the scheduling strategy according to the plurality of target functions and the processing order.

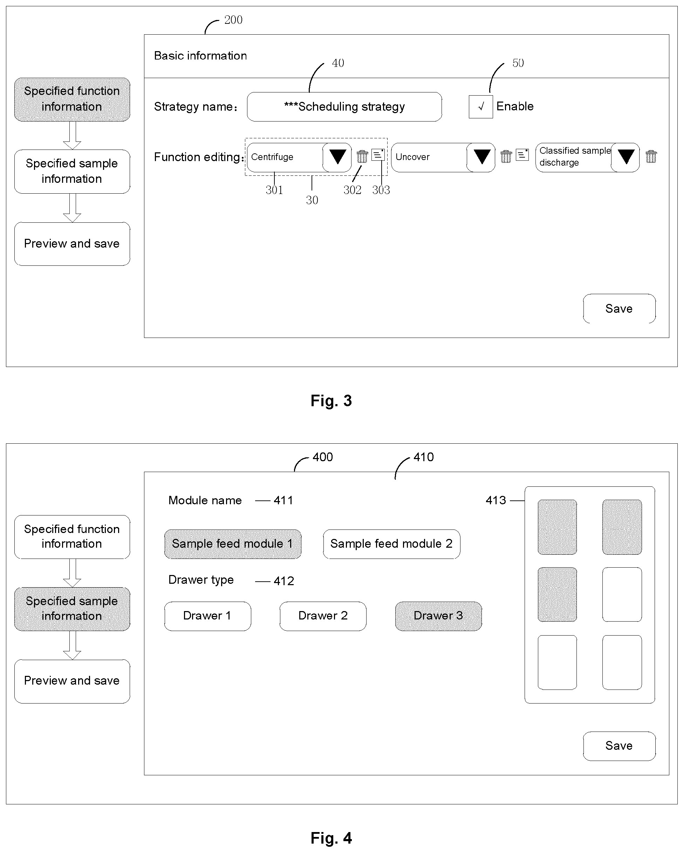

Alternatively, as shown in Fig. 3, a content configuration interface 200 may be used to display a plurality of setting components 30, which are sequentially arranged. In response to acquiring a plurality of target functions set in setting bars 301 of the plurality of setting components 30, a processing order of the target functions is acquired according to an order of the plurality of setting components 30.

As shown in Fig. 3, a centrifugation function is set at the setting bar 301 of the first setting component 30, an uncovering function is set at the setting bar of the second setting component 30, a classified sample discharge function is set at the setting bar of the third setting component 30, and therefore the processing order of the target functions of the scheduling strategy is: centrifugation -> uncovering -> classified sample discharge.

The processing content of the scheduling strategy is obtained according to the processing order of the plurality of target functions. For the processing strategy including the plurality of target functions, the scheduling strategy is used to instruct, according to the processing order, sequential scheduling of the target sample to the target function processing modules of the pipeline for processing. For example, the scheduling strategy shown in Fig. 3 is used to instruct, according to the order of centrifugation -> uncovering -> classified sample discharge, sequential scheduling of the target sample to the centrifugation processing module for centrifugation processing, to the uncovering processing module for uncovering processing, and to the sample discharge processing module for classified sample discharge processing according to the classified sample discharge rule.

In some embodiments, through the plurality of setting components, the processing content of the scheduling strategy can be flexibly configured as needed.

For the convenience of user configuration, the setting bars 301 of the setting components may be in the form of a drop-down box, which includes the processing functions provided by the pipeline. The user may perform selection from the drop-down box as needed to achieve settings, thereby improving setting convenience.

In some embodiments, the initially loaded processing content configuration interface may only include a limited number of setting components, and the limited number may be one or more. The last setting component 30 not only includes the setting bar 301 but also includes an add control 303. In response to more target functions being required according to user needs, setting components may be added by triggering the add control 303 of the setting component 30.

In some embodiments, the initially loaded processing content configuration interface may only include a limited number of setting components, and the limited number may be one or more. After configuring the target functions based on the existing setting components, the system automatically displays newly added setting components after the setting components, thereby facilitating user configuration of the target functions. In the method, after the user completely sets all the setting components currently displayed on the processing content configuration interface, the actively added new setting components facilitate user use, and if the user does not have requirements for configuring more target functions, no setting is required. For example, the initialized processing content configuration interface displays three setting components. After the user configures three target functions, a fourth setting component is automatically added. If the user has a requirement for configuring a fourth target function, setting may be performed at a setting bar of the fourth setting component, and after the fourth target function is configured in the fourth setting component, a fifth setting component is automatically added. If the user has no requirement for configuring the fourth target function, the user may click save to complete the configuration of the processing content.

In some embodiments, as shown in Fig. 3, the setting component 30 further includes a delete control 302. The user may delete the corresponding setting component and/or the target function set at the setting bar of the setting component by triggering the delete control 302.

In some embodiments, the setting component includes the add control and/or the delete control, to satisfy a plurality of configuration requirements of the user.

In some embodiments, to facilitate the management of the scheduling strategy, as shown in Fig. 3, the processing content configuration interface further includes a name setting area 40 for setting a configuration name of the scheduling strategy. Further, the processing content configuration interface further includes an enable switch 50, and by configuring the enable switch, the configuration strategy may be enabled or disabled.

In some embodiments, in response to the target function set at the setting bar of the setting component being a sample discharge related function, the add control of the setting component is hidden, or addition of a new setting component is stopped.

Alternatively, the sample discharge related function refers to a function related to pipeline sample discharge processing, which may typically include a general sample discharge function or the classified sample discharge function. For the samples, the sample discharge related function is the last function in pipeline processing. In response to the target function set at the setting bar of the setting component being the sample discharge related function, it may be considered that the last processing function of the scheduling strategy has been set. In this case, the add control of the setting component is hidden or addition of the new setting component is stopped, thereby avoiding mis-operations of the user and improving configuration intelligence and accuracy.

Alternatively, as shown in Fig. 3, hiding the add control of the setting component refers to hiding the add control of the setting component with the sample discharge related function set, to prevent the user from mistakenly operating to set other processing functions after the sample discharge related function.

Stopping the addition of a new setting component may include stopping the system from automatically adding the new setting component or stopping the response to user operations to add the new setting component, to prevent the user from mistakenly operating to set other processing functions after the sample discharge related function.

In some embodiments, the initialized processing content configuration interface may include a first setting component. After the user sets a first target function at a first setting bar of the first setting component, a second setting component is displayed on the processing content configuration interface by triggering an add control of the first setting component, and after the user sets a second target function at a second setting bar of the second setting component, if there is still a setting requirement, the user is facilitated in settings by triggering an add control of the second setting component. Accordingly, the multiple target functions may be set at the plurality of setting bars of the plurality of setting components.

The user can modify the target function by modifying the target function in the setting bar. The user can delete the target function by triggering the delete control of the setting component.

In some embodiments, in response to acquiring a trigger operation for the first setting bar of the first setting component, a first candidate function list is displayed; the first candidate function list includes the processing functions provided by the pipeline; the first target function selected from the first candidate function list is acquired; for an (N-1)^{th} setting component and an N^{th} setting component which are adjacent in the plurality of setting components, a trigger operation for an N^{th} setting bar of the N^{th} setting component is acquired, and according to an (N-1)^{th} target function set at an (N-1)^{th} setting bar of the (N-1)^{th} setting component, an N^{th} candidate function list is displayed, and an N^{th} target function selected from the N^{th} candidate function list is acquired, where N≥2; the N^{th} candidate function list includes other functions that the pipeline can continue to process the target sample after processing the target sample with the (N-1)^{th} function; and according to an order of the setting components, a processing order of the target functions is acquired.

The (N-1)^{th} setting component and the N^{th} setting component which are adjacent in the plurality of setting components refer to any two setting components adjacent in position, such as the first setting component and the second setting component, or the third setting component and the third setting component, which are determined according to the order of the setting components.

For the first setting component, in response to the user triggering the first setting bar for setting, the first candidate function list is displayed for the user to select from. The first candidate function list includes the processing functions provided by the pipeline, which are typically all processing functions provided by the pipeline.

Based on this, for the second setting component and subsequent setting components, any candidate processing functions for the N^{th} setting component from the (N-1)^{th} setting component and the N^{th} setting component which are adjacent are limited by a candidate function selected in the (N-1)^{th} setting component. Alternatively, the N^{th} candidate function list is displayed according to the (N-1)^{th} target function set at the (N-1)^{th} setting bar of the (N-1)^{th} setting component. The functions in the N^{th} candidate function list are other functions that the pipeline can continue to process the samples after processing the samples with the (N-1)^{th} function.

For example, a second candidate function list displayed at the second setting bar of the second setting component is determined according to the first target function selected in the first setting bar. Alternatively, functions in the second candidate function list are other functions that the pipeline can continue to process the target sample after processing the first target function.

In some embodiments, in response to setting two adjacent target functions, candidate functions in a drop-down box of the subsequent target function are determined according to the previous selected target function. Alternatively, the candidate functions in the drop-down box of the subsequent target function are other functions that the pipeline can continue to process the samples after performing the previous target function. For example, if the previous function is the centrifugation function, the candidate functions in the drop-down box of the subsequent setting component may include: serum quality analysis, uncovering, sample analysis, sample discharge, etc.

Due to certain requirements for the processing order of various functions in the pipeline, for example, the centrifugation function needs to be performed before the uncovering function. By setting the candidate processing functions of the N^{th} setting component to be limited from the candidate functions selected in the (N-1)^{th} setting component, the user can be prevented from setting an order of the target functions that contradicts the processing function order of the pipeline.

In some embodiments, the step of determining a target sample applying the scheduling strategy includes: determining the target sample applying the scheduling strategy according to at least one of a sample feed position, patient information associated with a sample, a liquid type, a sample type, sample container information, and test item information.

In some embodiments, the user may set screening conditions from at least one dimension among the sample feed position, the patient information associated with the sample, the liquid type, the sample type, the sample container information, and the test item information, to determine the target sample applying the scheduling strategy.

Further, to facilitate user settings, an application object configuration interface may be provided. The application object configuration interface at least includes at least one of a sample feed position setting component, a patient information setting component, a liquid type setting component, a sample type setting component, a container information setting component, and an item information setting component. The sample screening conditions configured in at least one of the sample feed position setting component, the patient information setting component, the liquid type setting component, the sample type setting component, the container information setting component, and the item information setting component are acquired. The target sample applying the scheduling strategy is determined according to the sample screening conditions.

The sample feed position setting component may be configured to set sample feed position screening conditions. The sample feed position screening conditions may be set from three aspects of the sample feed processing module, the drawer, and the sample box, to specify a sample at the specific sample feed position as the target sample applying the scheduling strategy.

The patient information setting component may be configured to set patient information screening conditions, to determine a sample about patient information that conforms to setting conditions as the target sample applying the scheduling strategy. The patient information may include a patient gender, an age, a diagnosing department, etc.

The liquid type setting component may be configured to set liquid type screening conditions, to specify a sample of a specific liquid type as the target sample applying the scheduling strategy. The liquid types may include whole blood, serum, plasma, etc. In response to the liquid type being the serum, it indicates that centrifugation has already been performed, and therefore there is no need to configure the centrifugation function during configuring the scheduling strategy.

The sample type setting component may be configured to set sample type screening conditions, to specify a sample of a specific sample type as the target sample applying the scheduling strategy. Sample types may include emergency samples or routine samples, thereby allowing for the configuration of scheduling strategies for the emergency samples or the routine samples.

A item information setting component may be configured to set test item screening conditions, to specify a sample including a specific test item as the target sample applying the scheduling strategy. Test items refer to items that need to be tested in the sample, such as a liver function test item or a kidney function test item. Therefore, scheduling strategies may be configured for different test items.

The container information setting component may be configured to set container information screening conditions, to specify a sample within a specific container as the target sample applying the scheduling strategy. The sample container information refers to information about a container loading samples, including a diameter and a height of the sample container, whether the sample container has a container cover or not, a sample loading capacity, etc. Therefore, different scheduling strategies may be configured for different sample containers. For example, for samples loaded in a sample container without a container cover, there is no need for scheduling to an uncovering module, and a scheduling strategy without an uncovering function may be configured for the type of samples.

Sample screening conditions of the target sample may be limited from any dimension among the sample feed position, the patient information associated with the sample, the liquid type, the sample type, the sample container information, and the test item information, or may be limited from a combination of any two dimensions among the sample feed position, the patient information associated with the sample, the liquid type, the sample type, the sample container information, and the test item information, such as the sample feed position and the sample type, thereby setting the sample screening conditions to determine the target sample. For another example, a limitation may be made from the sample feed position, the patient information associated with the sample, the liquid type, the sample type, the sample container information, and the test item information, to set the sample screening conditions to determine the target sample.

In some embodiments, by setting the sample screening conditions from a plurality of dimensions, the target sample applying the scheduling strategy can be flexibly selected, thereby improving scheduling intelligence of the pipeline.

In some embodiments, a method for acquiring sample screening conditions configured at the sample feed position setting component includes: displaying the sample feed position setting component and acquiring a sample feed position attribute set through the sample feed position setting component, where the sample feed position attribute includes at least one of a target drawer and a target sample box; and determining sample screening conditions set from the dimension of the sample feed position according to the sample feed position attribute.

In some embodiments, by displaying the sample feed position setting component on the application object configuration interface, the user may set the sample feed position attribute through the sample feed position setting component. The sample feed position attribute includes at least one of the target drawer and the target sample box. In some embodiments, if the pipeline only includes one sample feed processing module, the sample feed position attribute may include the target drawer and the target sample box, or the sample feed position attribute may also only include the target drawer. In some embodiments, if the pipeline includes a plurality of sample feed processing modules, the sample feed position attribute may include a target sample feed processing module, the target drawer, and the target sample box, or the sample feed position attribute may also only include the target sample feed processing module and the target drawer

According to the sample feed position attribute, the sample screening condition is determined from the dimension of the sample feed position, and according to the screening condition, the target sample may be screened according to the sample feed position attribute. For example, if the sample feed position attribute includes a sample feed processing module 1, a drawer 1, and a sample box 1, a sample in the sample box 1 in the drawer 1 of the sample feed processing module 1 may be determined as the target sample. For another example, if the sample feed position attribute includes the sample feed processing module and the drawer 1, all samples in the drawer 1 of the sample feed processing module 1 may be determined as target samples.

In some embodiments, by arranging the sample feed position setting component, the user may be facilitated to set the sample screening conditions from the dimension of the sample feed position, to determine the target sample.

Alternatively, as shown in Fig. 4, if the pipeline includes a plurality of sample feed processing modules, a sample feed position setting component 410 on the application object configuration interface includes a sample feed module setting area 411, a drawer setting area 412, and a sample box setting area 413.

The displaying the sample feed position setting component and acquiring a sample feed position attribute set through the sample feed position setting component includes: displaying the sample feed module setting area, the drawer setting area, and the sample box setting area, and displaying sample feed module icons in the sample feed module setting area corresponding to the sample feed processing modules of the pipeline in number; after acquiring a selected sample feed module icon, determining a target sample feed processing module, and displaying drawer icons in the drawer setting area corresponding to drawers of the target sample feed processing module in number; a layout of the drawer icons corresponding to an actual layout of the drawers in the target sample feed processing module; after acquiring a selected drawer icon, determining a target drawer to obtain a sample feed position attribute, where the sample feed position attribute includes the target sample feed processing module and the target drawer, or after acquiring a selected drawer icon, determining a target drawer, and displaying sample box icons in the sample box setting area corresponding to sample boxes in the target drawer in number; a layout of the sample box icons corresponding to an actual layout of the sample boxes in the target drawer; and after acquiring a selected sample box icon, determining a target sample box to obtain a sample feed position attribute, where the sample feed position attribute includes the target sample feed processing module, the target drawer, and the target sample box.

Alternatively, as shown in Fig. 4, the sample feed module icons corresponding to the sample feed processing modules of the pipeline in number are displayed in the sample feed module setting area 411 of the sample feed position setting component 410. For example, the pipeline includes two sample feed processing modules, and two sample feed module icons are displayed in the sample feeding module setting area 411 for user selection. After the user selects a sample feed module icon, a target sample feed processing module is determined, and drawer icons corresponding to drawers of the target sample feed processing module in number are displayed in the drawer setting area 412. For example, the target sample feed processing module includes three drawers, and three drawer icons are displayed in the drawer setting area 412 for user selection. For ease of user understanding, the layout of the drawer icons corresponds to the actual layout of the drawers in the target sample feed processing module, thereby allowing the user to match the drawer icons with physical positions of the drawers and facilitating user selection.

After the user selects a drawer icon, a target drawer is determined, and sample box icons corresponding to sample boxes in the target drawer in number are displayed in the sample box setting area 413. For example, the drawer has six sample boxes, and six sample box icons are displayed in the sample box setting area 413 for user selection. For ease of user understanding, the layout of the sample box icons corresponds to the actual layout of the sample boxes in the target drawer, thereby allowing the user to match the sample box icons with actual physical positions of the sample boxes and facilitating user selection.

If the user selects the target drawer and then chooses to save, the sample feed position attribute is obtained according to the target sample feed processing module and the target drawer. The sample feed position attribute includes the target sample feed processing module and the target drawer, thereby determining all samples in the target drawer of the target sample feed processing module as target samples.

After the selected sample box icon is acquired, the target sample box is determined, and accordingly the sample feed position attribute includes: the target sample feed processing module, the target drawer, and the target sample box. Therefore, samples in the target sample box in the target drawer of the target sample feed processing module may be determined as target samples.

In some embodiments, by providing the setting component that corresponds to a hierarchical attribute of an actual sample feed position, the configuration convenience is improved. A visual configuration method that can achieve mapping to an actual physical position is provided in a drawer and sample box configuration process, thereby improving configuration convenience and accuracy.

In some embodiments, if the pipeline includes one sample feed processing module, the sample feed position setting component includes the drawer setting area and the sample box setting area.

The displaying the sample feed position setting component and acquiring a sample feed position attribute set through the sample feed position setting component includes: displaying the drawer setting area and the sample box setting area, and displaying drawer icons in the drawer setting area corresponding to drawers of the sample feed processing module in number; a layout of the drawer icons corresponding to an actual layout of the drawers in the sample feed processing module; after acquiring a selected drawer icon, determining a target drawer to obtain a sample feed position attribute, where the sample feed position attribute includes the target drawer, or after acquiring a selected drawer icon, determining a target drawer, and displaying sample box icons in the sample box setting area corresponding to sample boxes in the target drawer in number; a layout of the sample box icons corresponding to an actual layout of the sample boxes in the target drawer; and after acquiring a selected sample box icon, determining a target sample box to obtain a sample feed position attribute, where the sample feed position attribute includes the target drawer and the target sample box.

For a scenario with only one sample feed processing module, a sample feed processing module selecting process may be omitted. The sample feed position attribute is determined through the drawer configuration and the sample box configuration, or is determined through the drawer configuration.

A method for configuring a pipeline scheduling strategy according to some embodiments is shown from Fig. 3 to Fig. 5.

Firstly, a strategy configuration interface is provided, and may be divided into a navigation area and a setting area.

The navigation area is used to display setting navigation bars, and the setting area is used to display a relevant configuration interface.

Three navigation bars are included, which are respectively configuration of specified function information, configuration of specified sample information, and preview and save.

In the configuration stage of the specified function information, a processing content configuration interface 200 is displayed in the setting area. The processing content configuration interface 200 may be configured to display at least one setting component 30. The setting component 30 includes the setting bar 301, the add control 303, and the delete control 302.

The initially loaded processing content configuration interface may only include a limited number of setting components, and the limited number may be one or more. In response to requiring more target functions according to user needs, setting components may be added by triggering the add control 303 of the setting component 30. The user sets target functions through the setting bar of the setting component, and may delete the corresponding setting component and the target function set at the setting bar of the setting component by triggering the delete control 302.

The processing content configuration interface further includes the name setting area 40 for setting a configuration name of the scheduling strategy. Further, the processing content configuration interface further includes the enable switch 50, and by configuring the enable switch, the configuration strategy may be enabled or disabled.

In response to acquiring a plurality of target functions set at the setting bars of the plurality of setting components, a processing order of the target functions is acquired, and a processing content of the scheduling strategy is obtained according to the plurality of target functions and the processing order. In the configuration stage of the specified function information, the configuration of the processing content of the scheduling strategy is completed.

In the configuration stage of the specified sample information, a target sample applying the scheduling strategy may be configured by specifying a sample feed position. Alternatively, an application object configuration interface 400 is displayed in the setting area and may display the sample feed position setting component 410. The sample feed position setting component 410 includes the sample feed module setting area 411, the drawer setting area 412, and the sample box setting area 413.

Sample feed module icons corresponding to sample feed processing modules of a pipeline in number are displayed in the sample feed module setting area 411 of the sample feed position setting component 410. For example, the pipeline includes two sample feed processing modules, and two sample feed module icons are displayed in the sample feeding module setting area 411 for user selection. After the user selects a sample feed module icon, a target sample feed processing module is determined, and drawer icons corresponding to drawers of the target sample feed processing module in number are displayed in the drawer setting area 412. For example, the target sample feed processing module includes three drawers, and three drawer icons are displayed in the drawer setting area 412 for user selection. For ease of user understanding, a layout of the drawer icons corresponds to an actual layout of the drawers in the target sample feed processing module, thereby allowing the user to match the drawer icons with physical positions of the drawers and facilitating user selection.

After the user selects a drawer icon, a target drawer is determined, and sample box icons corresponding to sample boxes in the target drawer in number are displayed in the sample box setting area 413. For example, the drawer has six sample boxes, and six sample box icons are displayed in the sample box setting area 413 for user selection. For ease of user understanding, a layout of the sample box icons corresponds to an actual layout of the sample boxes in the target drawer, thereby allowing the user to match the sample box icons with actual physical positions of the sample boxes and facilitating user selection.

Therefore, the user may set the target sample feed processing module, the target drawer, and the target sample box through the sample feed position setting component, to determine a sample in the target sample box in the target drawer of the target sample feed processing module as a target sample. In the configuration stage of the specified sample information, the configuration of the target sample applying the scheduling strategy is completed.

In a preview stage, the user may view a specific content of the configured scheduling strategy, including functions involved in the processing content of the scheduling strategy and an application object. After the preview is correct, the user clicks save to complete the configuration of the scheduling strategy.

In some embodiments, through the setting components, the user can conveniently set the target functions of the scheduling strategy. Through the sample feed position setting component, the user can conveniently specify a sample at a sample feed position as the target sample, thereby achieving the configuration of the scheduling strategy. In the configuration process of the scheduling strategy, the convenience and accuracy of the configuration are improved by providing limitations on the order of the target functions, visual configuration, and other means. Through the configuration method of the scheduling strategy, different target functions or combinations can be configured for different target samples, thereby achieving intelligent scheduling of the pipeline.

It should be understood that although the steps in flowcharts related to the above embodiments are shown sequentially according to the direction of arrows, these steps are not necessarily performed in an order indicated by the arrows. Unless explicitly stated in this specification, there are no strict order limitations on the execution of these steps, and these steps may be performed in another order. In addition, at least some steps in the flowcharts involved in the above embodiments may include a plurality of steps or a plurality of stages, and these steps or stages are not necessarily performed at the same moment, and may be performed at different moments. These steps or stages are not necessarily performed in sequence, and may be performed in turns or alternately with other steps, or at least a part of steps or stages in the other steps.

Based on the same inventive concept, some embodiments of this application further provides an apparatus for configuring a pipeline scheduling strategy to implement the above method for configuring a pipeline scheduling strategy. An implementation solution provided by the apparatus for solving the problems is similar to an implementation solution recorded in the above method. Therefore, for specific limitations in one or more embodiments of the apparatus for configuring the pipeline scheduling strategy provided below, reference may be made to the limitations on the method for configuring a pipeline scheduling strategy as above, which will not be repeated herein.

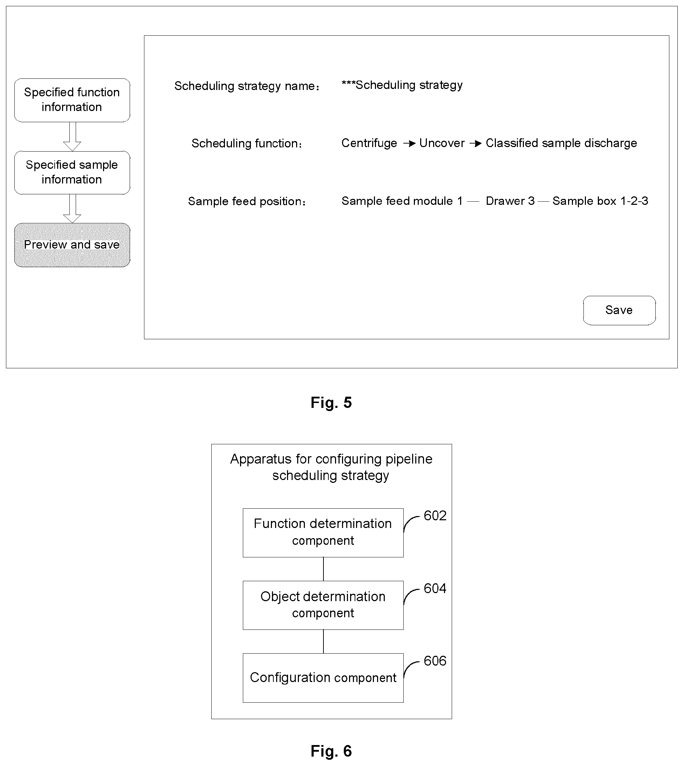

In some embodiments, as shown in Fig. 6, an apparatus for configuring a pipeline scheduling strategy is provided, and includes:
a function determination component 602, configured to determine a processing content of a scheduling strategy, where the processing content of the scheduling strategy includes a target function determined from processing functions provided by the pipeline;
an object determination component 604, configured to determine a target sample applying the scheduling strategy; and
a configuration component 606, configured to configure the scheduling strategy according to the processing content and the target sample, where the scheduling strategy is used to instruct a processing module with the target function on the pipeline to process the target sample.

In some embodiments, the processing content includes at least two target functions, and the processing content further includes: a processing order of the at least two target functions.

The step of configuring the scheduling strategy according to the processing content and the target sample includes:
configuring the scheduling strategy according to the target functions, the processing order
of the target functions, and the target sample, where the scheduling strategy is used to instruct, according to the processing order, sequential scheduling of the target sample to the processing modules with the target functions on the pipeline for processing.

In some embodiments, the step of determining a processing content of a scheduling strategy includes:
providing a processing content configuration interface, where the processing content configuration interface includes at least one setting component, and the setting component at least includes a setting bar; and

In response to a target function set at the setting bar of the first or last setting component being a classified sample discharge function, obtaining the processing content of the scheduling strategy according to the classified sample discharge function, where the scheduling strategy is used to instruct a sample discharge processing module on the pipeline to perform sample discharge processing on the target sample according to a classified sample discharge rule.

In some embodiments, the step of determining a processing content of a scheduling strategy includes:
providing a processing content configuration interface, where the processing content configuration interface includes at least one setting component, and the setting component at least includes a setting bar;
in response to acquiring a plurality of target functions set at the setting bars of the plurality of setting components, acquiring a processing order of the target functions; and
obtaining the processing content of the scheduling strategy according to the plurality of target functions and the processing order.

In some embodiments, the method further includes:
the setting component further including an add control, and in response to triggering the add control of the setting component, displaying a newly added setting component behind the setting component;
alternatively,
after setting a target function at the setting bar of the setting component, displaying a newly added setting component behind the setting component.
In some embodiments, the method further includes:
in response to the target function set at the setting bar of the setting component being a sample discharge related function, hiding the add control behind the setting component,
or stopping addition of a new setting component.

In some embodiments, the setting component further includes a delete control. The method further includes:
deleting the corresponding setting component and/or the target function set at the setting bar of the setting component in response to triggering the delete control.

In some embodiments, the step that in response to acquiring a plurality of target functions set at the setting bars of the plurality of setting components, a processing order of the target functions is acquired includes:
in response to acquiring a trigger operation for a first setting bar of a first setting component, displaying a first candidate function list, where the first candidate function list includes the processing functions provided by the pipeline;
acquiring a first target function selected from the first candidate function list;
for an (N-1)^{th} setting component and an N^{th} setting component which are adjacent in the plurality of setting components, acquiring a trigger operation for an N^{th} setting bar of the N^{th} setting component, and according to an (N-1)^{th} target function set at an (N-1)^{th} setting bar of the (N-1)^{th} setting component, displaying an N^{th} candidate function list, and acquiring an N^{th} target function selected from the N^{th} candidate function list, where N≥2, and the N^{th}candidate function list includes other functions that the pipeline can continue to process the target sample after processing the target sample with the (N-1)^{th} function; and
according to an order of the setting components, acquiring a processing order of the target functions.

In some embodiments, the step of determining a target sample applying the scheduling strategy includes: determining the target sample applying the scheduling strategy according to at least one of a sample feed position, patient information associated with a sample, a liquid type, a sample type, sample container information, and test item information.

In some embodiments, the determining the target sample applying the scheduling strategy according to at least one of a sample feed position, patient information associated with a sample, a liquid type, a sample type, sample container information, and test item information includes:
providing an application object configuration interface, where the application object configuration interface includes at least one of a sample feed position setting component,
a patient information setting component, a liquid type setting component, a sample type setting component, a container information setting component, and an item information setting component;
acquiring sample screening conditions configured in at least one of the sample feed position setting component, the patient information setting component, the liquid type setting component, the sample type setting component, the container information setting component, and the item information setting component; and
determining the target sample applying the scheduling strategy according to the sample screening conditions.

In some embodiments, a method for acquiring sample screening conditions configured at the sample feed position setting component includes:
displaying the sample feed position setting component and acquiring a sample feed position attribute set through the sample feed position setting component, where the sample feed position attribute includes at least one of a target drawer and a target sample box; and
determining sample screening conditions set from the dimension of the sample feed position according to the sample feed position attribute.

In some embodiments, if the pipeline includes a plurality of sample feed processing modules, the sample feed position setting component includes a sample feed module setting area, a drawer setting area, and a sample box setting area.

The displaying the sample feed position setting component and acquiring a sample feed position attribute set through the sample feed position setting component includes:
displaying the sample feed module setting area, the drawer setting area, and the sample box setting area, and displaying sample feed module icons in the sample feed module setting area corresponding to the sample feed processing modules of the pipeline in number;
after acquiring a selected sample feed module icon, determining a target sample feed processing module, and displaying drawer icons in the drawer setting area corresponding to drawers of the target sample feed processing module in number; a layout of the drawer icons corresponding to an actual layout of the drawers in the target sample feed processing module;
after acquiring a selected drawer icon, determining a target drawer to obtain a sample feed position attribute, where the sample feed position attribute includes the target sample feed processing module and the target drawer, or after acquiring a selected drawer icon,
determining a target drawer, and displaying sample box icons in the sample box setting area corresponding to sample boxes in the target drawer in number; a layout of the sample box icons corresponding to an actual layout of the sample boxes in the target drawer; and
after acquiring a selected sample box icon, determining a target sample box to obtain a sample feed position attribute, where the sample feed position attribute includes the target sample feed processing module, the target drawer, and the target sample box.

Alternatively, if the pipeline includes one sample feed processing module, the sample feed position setting component includes the drawer setting area and the sample box setting area; and
the displaying the sample feed position setting component and acquiring a sample feed position attribute set through the sample feed position setting component includes:
displaying the drawer setting area and the sample box setting area, and displaying drawer icons in the drawer setting area corresponding to drawers of the sample feed processing module in number; a layout of the drawer icons corresponding to an actual layout of the drawers in the sample feed processing module;
after acquiring a selected drawer icon, determining a target drawer to obtain a sample feed position attribute, where the sample feed position attribute includes the target drawer, or after acquiring a selected drawer icon, determining a target drawer, and displaying sample box icons in the sample box setting area corresponding to sample boxes in the target drawer in number; a layout of the sample box icons corresponding to an actual layout of the sample boxes in the target drawer; and after acquiring a selected sample box icon, determining a target sample box to obtain a sample feed position attribute, where the sample feed position attribute includes the target drawer and the target sample box.

Various components in the above apparatus for configuring the pipeline scheduling strategy may be all or partly implemented by software, hardware, and a combination thereof. The foregoing components may be built in or independent of a processor of a computer device in a form of hardware, or may be stored in a memory of the computer device in a form of software, for the processor to invoke to execute operations corresponding to the foregoing components.

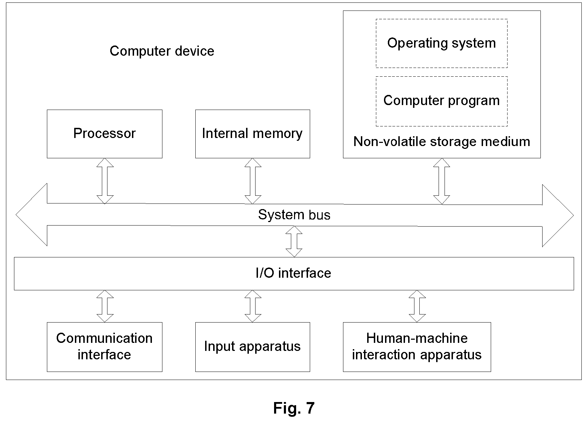

In some embodiments, a pipeline control terminal is provided, with an internal structure illustrated in Fig. 7. The pipeline control terminal includes a processor, a memory, and a non-volatile storage medium, which are connected through a system bus, as well as a communication interface, an input apparatus, and a human-machine interaction apparatus, which are connected through an I/O interface. The processor of the pipeline control terminal is configured to provide computation and control capabilities. The memory of the pipeline control terminal includes a non-volatile storage medium and an internal memory. The non-volatile storage medium stores an operating system, a computer program, and a database. The internal memory provides an environment for running of the operating system and the computer program in the non-volatile storage medium. The communication interface of the pipeline control terminal is configured to communicate with an external terminal in a wired or wireless mode. The wireless mode may be implemented through wireless fidelity (WIFI), a mobile cellular network, near field communication (NFC), or another technology. The computer program, when executed by the processor, implements the method for configuring a pipeline scheduling strategy. The human-machine interaction apparatus of the pipeline control terminal may be a liquid crystal display screen or an electronic ink display screen. The input apparatus of the pipeline control terminal may be a touch layer covering a display screen, or may be a button, a trackball, or a touchpad set on a computer device casing, or may also be an external keyboard, a touchpad, a mouse, or the like.

Those skilled in the art should understand that the structure shown in Fig. 7 is only a block diagram of a partial structure relevant to the solutions of this application, and does not constitute a limitation on the computer device to which the solutions of this application are applied. The specific computer device may include more or fewer components than those shown in the figure, or combine certain components, or have different arrangements of components.

In some embodiments, a pipeline control terminal is provided, and includes a memory and a processor. The memory has a computer program stored therein. The processor, when executing the computer program, implements the steps of the method for configuring a pipeline scheduling strategy according to the above embodiments.

In some embodiments, a computer-readable storage medium is provided and has a computer program stored therein. The computer program, when executed by a processor, implements the steps of the method for configuring a pipeline scheduling strategy according to the above embodiments.

In some embodiments, a computer program product is provided, including a computer program. The computer program, when executed by a processor, implements the steps of the method for configuring a pipeline scheduling strategy according to the above embodiments.

Those of ordinary skill in the art should understand that all or some of the processes in the above embodiment methods may be implemented by instructing relevant hardware through the computer program. The computer program may be stored in a non-volatile computer-readable storage medium. The computer program, when executed, may include the processes of the above method embodiments. Any reference to the memory, the database, or another medium used in the embodiments provided in this application may include at least one of non-volatile and volatile memories. The non-volatile memory may include a read-only memory (ROM), a magnetic tape, a floppy disk, a flash memory, an optical memory, a high-density embedded non-volatile memory, a resistive random access memory (ReRAM), a magnetoresistive random access memory (MRAM), a ferroelectric random access memory (FRAM), a phase change memory (PCM), a graphene memory, etc. The volatile memory may include a random access memory (RAM) or an external cache memory. As an illustration rather than a limitation, the RAM is available in various forms, such as a static random access memory (SRAM) or a dynamic random access memory (DRAM). The database involved in the embodiments provided in this application may include at least one of a relational database and a non-relational database. The non-relational database may include a blockchain-based distributed database, or the like, which is not limited herein. The processor involved in the embodiments provided in this application may be a general-purpose processor, a central processing unit, a graphics processing unit, a digital signal processor, a programmable logic device, a quantum computing-based data processing logic device, or the like, which is not limited herein.

Various technical features of the foregoing embodiments may be combined at will. For brevity of the description, it is unnecessary to describe all possible combinations of the various technical features of the foregoing embodiments, however, the combinations of these technical features should be considered within the scope recorded by this specification as long as there is no contradiction.

The foregoing embodiments merely express several implementations of this application. The descriptions thereof are relatively specific and detailed, but should not be understood as limitations to the patent scope of this application. It should be noted that for those of ordinary skill in the art, several transformations and improvements may also be made without departing from the idea of this application. These transformations and improvements belong to the scope of protection of this application. Therefore, the scope of protection of this application shall be subject to the appended claims.

## Claims

1. A method for configuring a pipeline scheduling strategy, comprising:
determining a processing content of a scheduling strategy, wherein the processing content of the scheduling strategy comprises a target function determined from processing functions provided by a pipeline;
determining a target sample applying the scheduling strategy; and
configuring the scheduling strategy according to the processing content and the target sample, wherein the scheduling strategy is used to instruct a processing module with the target function on the pipeline to process the target sample.

2. The method as claimed in claim 1, wherein the processing content comprises at least two target functions, and the processing content further comprises a processing order of the at least two target functions; and the configuring the scheduling strategy according to the processing content and the target sample comprises:
configuring the scheduling strategy according to the target functions, the processing order of the target functions, and the target sample, wherein the scheduling strategy is used to instruct, according to the processing order, sequential scheduling of the target sample to processing modules with the target functions on the pipeline for processing.

3. The method as claimed in claim 1, wherein the determining the processing content of the scheduling strategy comprises:
providing a processing content configuration interface, wherein the processing content configuration interface comprises at least one setting component, and the setting component at least comprises a setting bar; and
in response to the target function set at the setting bar of the first setting component or last setting component being a classified sample discharge function, obtaining the processing content of the scheduling strategy according to the classified sample discharge function, wherein the scheduling strategy is used to instruct a sample discharge processing module on the pipeline to perform sample discharge processing on the target sample according to a classified sample discharge rule.

4. The method as claimed in claim 1, wherein the determining the processing content of the scheduling strategy comprises:
providing a processing content configuration interface, wherein the processing content configuration interface comprises at least one setting component, and the setting component at least comprises a setting bar; and
in response to acquiring a plurality of target functions set at the setting bars of the plurality of setting components, acquiring a processing order of the target functions; and
obtaining the processing content of the scheduling strategy according to the plurality of target functions and the processing order.

5. The method as claimed in claim 3 or 4, further comprising:
the setting component further comprising an add control, and in response to triggering the add control of the setting component, displaying a newly added setting component behind the setting component;
or,
after setting the target function at the setting bar of the setting component, displaying a newly added setting component behind the setting component.

6. The method as claimed in claim 5, further comprising:
in response to the target function set at the setting bar of the setting component being a sample discharge related function, hiding the add control behind the setting component, or stopping addition of a new setting component.

7. The method as claimed in claim 3 or 4, wherein the setting component further comprises a delete control, and the method further comprises:
In response to triggering the delete control ,deleting at least one of the following: the setting component or the target function set at the setting bar of the setting component.

8. The method as claimed in claim 3 or 4, wherein the processing content configuration interface further comprises a name setting area for setting a configuration name of the scheduling strategy.

9. The method as claimed in claim 3 or 4, wherein the processing content configuration interface further comprises an enable switch, the scheduling strategy is enabled or disabled by configuring the enable switch.

10. The method as claimed in claim 4, wherein the in response to acquiring the plurality of target functions set at the setting bars of the plurality of setting components, acquiring the processing order of the target functions comprises:
in response to acquiring a trigger operation for a first setting bar of a first setting component, displaying a first candidate function list, wherein the first candidate function list comprises processing functions provided by the pipeline;
acquiring a first target function selected from the first candidate function list;
for an (N-1)^{th} setting component and an N^{th} setting component which are adjacent in the plurality of setting components, acquiring a trigger operation for an N^{th} setting bar of the N^{th} setting component, and according to an (N-1)^{th} target function set at an (N-1)^{th} setting bar of the (N-1)^{th} setting component, displaying an N^{th} candidate function list, and acquiring an N^{th} target function selected from the N^{th} candidate function list, wherein N≥2, and the N^{th} candidate function list comprises other functions that the pipeline can continue to process the target sample after processing the target sample with the (N-1)^{th} target function; and
according to an order of the setting components, acquiring a processing order of the target functions.

11. The method as claimed in any one of claims 1 to 4 or claim 10, wherein the determining the target sample applying the scheduling strategy comprises:
determining the target sample applying the scheduling strategy according to at least one of a sample feed position, patient information associated with a sample, a liquid type, a sample type, sample container information, and test item information.

12. The method as claimed in claim 11, wherein the determining the target sample applying the scheduling strategy according to at least one of the sample feed position, patient information associated with the sample, the liquid type, the sample type, sample container information, and test item information comprises:
providing an application object configuration interface, wherein the application object configuration interface comprises at least one of a sample feed position setting component, a patient information setting component, a liquid type setting component, a sample type setting component, a container information setting component, and an item information setting component;
acquiring sample screening conditions configured in at least one of the sample feed position setting component, the patient information setting component, the liquid type setting component, the sample type setting component, the container information setting component, and the item information setting component; and
determining the target sample applying the scheduling strategy according to the sample screening conditions.

13. The method as claimed in claim 12, wherein a method for acquiring the sample screening conditions configured at the sample feed position setting component comprises:
displaying the sample feed position setting component and acquiring a sample feed position attribute set through the sample feed position setting component, wherein the sample feed position attribute comprises at least one of a target drawer and a target sample box; and
determining sample screening conditions set from the dimension of the sample feed position according to the sample feed position attribute.

14. The method as claimed in claim 13, wherein, in response to the pipeline comprising a plurality of sample feed processing modules, the sample feed position setting component comprises a sample feed module setting area, a drawer setting area, and a sample box setting area; and the displaying the sample feed position setting component and acquiring a sample feed position attribute set through the sample feed position setting component comprises:
displaying the sample feed module setting area, the drawer setting area, and the sample box setting area, and displaying sample feed module icons in the sample feed module setting area corresponding to the sample feed processing modules of the pipeline in number;
after acquiring a selected sample feed module icon, determining a target sample feed processing module, and displaying drawer icons in the drawer setting area corresponding to drawers of the target sample feed processing module in number; a layout of the drawer icons corresponding to an actual layout of the drawers in the target sample feed processing module;
after acquiring a selected drawer icon, determining a target drawer to obtain the sample feed position attribute, wherein the sample feed position attribute comprises the target sample feed processing module and the target drawer; or after acquiring a selected drawer icon, determining a target drawer, and displaying sample box icons in the sample box setting area corresponding to sample boxes in the target drawer in number, a layout of the sample box icons corresponding to an actual layout of the sample boxes in the target drawer; and after acquiring a selected sample box icon, determining a target sample box to obtain the sample feed position attribute, wherein the sample feed position attribute comprises the target sample feed processing module, the target drawer, and the target sample box;
or,
in response to the pipeline comprising one sample feed processing module, the sample feed position setting component comprises the drawer setting area and the sample box setting area; and the displaying the sample feed position setting component and acquiring a sample feed position attribute set through the sample feed position setting component comprises:
displaying the drawer setting area and the sample box setting area, and displaying drawer icons in the drawer setting area corresponding to drawers of the sample feed processing module in number; a layout of the drawer icons corresponding to an actual layout of the drawers in the sample feed processing module;
after acquiring a selected drawer icon, determining a target drawer to obtain the sample feed position attribute, wherein the sample feed position attribute comprises the target drawer; or after acquiring a selected drawer icon, determining a target drawer, and displaying sample box icons in the sample box setting area corresponding to sample boxes in the target drawer in number, a layout of the sample box icons corresponding to an actual layout of the sample boxes in the target drawer; and after acquiring a selected sample box icon, determining a target sample box to obtain the sample feed position attribute, wherein the sample feed position attribute comprises the target drawer and the target sample box.

15. A pipeline system, comprising a track, various function processing modules connected through the track, and a pipeline control terminal, wherein the processing modules and the track are respectively in communication connection with the pipeline control terminal, the pipeline control terminal comprises a memory and a processor, wherein the memory has a computer program stored therein, and the processor is configured to execute the computer program to implement the steps of the method as claimed in any one of claims 1 to 14.
